# EUROPEAN PATENT APPLICATION

(11) **EP 4 368 294 A1**
(43) Date of publication of application: **15.05.2024**
(21) Application number: 22836762.9
(22) Date of filing: 28.06.2022
(51) Int. Cl.: B04B 1/00, B04B 7/12, B04B 15/00, B04B 11/02, C12M 1/00, C12M 3/00, C12M 1/12, C12M 1/10, C12M 1/04, C12M 1/02, C12N 5/00

(54) **CELL CENTRIFUGATION APPARATUS AND CELL CENTRIFUGATION, CLEANING, AND CULTURING METHODS**

(30) Priority: 06.07.2021 CN 202110761903; 06.07.2021 CN 202121524433 U
(71) Applicant: Shenzhen Cellbri Bio-Innovation Technology Co., Ltd., Shenzhen, Guangdong 518107 (CN)
(72) Inventor: GUO, Xiaoliang, Shenzhen, Guangdong 518107 (CN); SHANG, Yuanfang, Shenzhen, Guangdong 518107 (CN); YAO, Jialin, Shenzhen, Guangdong 518107 (CN); ZHANG, Jinxin, Shenzhen, Guangdong 518107 (CN); LIU, Fujing, Shenzhen, Guangdong 518107 (CN)
(74) Representative: Herrero & Asociados, S.L.
(86) International application number: PCT/CN2022/101748
(87) International publication number: WO 2023/280008

(57) **Abstract**

The present application disclosed a cell centrifugation apparatus and a cell centrifugation, washing and culture method. The cell centrifugation apparatus comprises a centrifuge chamber (1) and a liquid pushing plate (2); the centrifuge chamber (1) has a rotation axis, and the centrifuge chamber (1) is used to connected with a rotation drive mechanism (4) and rotate around its rotation axis under drive of the rotation drive mechanism (4), so as to centrifuge a blood sample contained in the centrifuge chamber (1); and one or more liquid pushing plates (2) are arranged in the centrifuge chamber (1), when the centrifuge chamber (1) rotates, the liquid pushing plate (2) is used to push the blood sample to rotate synchronously along the same rotation direction as the centrifuge chamber (1).

## Description

### Cross reference to related applications

The present application claims the benefits of Chinese Patent Application No. 202110761903.1 filed on July 6, 2021, titled "Cell centrifugation apparatus and cell centrifugation, washing and culture method", and China Patent Application No.202121524433.9 filed on July 6, 2021, titled "Cell centrifugation apparatus", the contents of which are incorporated herein by reference in their entirety.

### Technical field

The application relates to the technical field of medical instruments, in particular to a cell centrifugation apparatus and a cell centrifugation, washing and culture method.

### Background

In the field of cell medicine, cells collected in vivo are often cultured and inoculated into patients. In order to ensure the therapeutic effect of cells collected and cultured from living body, it is necessary to sort the peripheral blood mononuclear cells (PBMC), and at the same time, it is necessary to remove impurities and culture medium during cell culture and concentrate the cells to a cell concentration suitable for treatment. The method of peripheral blood mononuclear cells is density gradient centrifugation. That is, different particles in blood samples are settled at a certain rate under the action of centrifugal force, and finally zones are formed in different regions to achieve the purpose of separation.

For the centrifugal process of the existing centrifugal equipment, the inner side wall of the centrifugal equipment uses viscous resistance to drive the outer liquid near the inner side wall to rotate, and the outer liquid uses viscous resistance to drive the inner liquid away from the inner side wall to rotate, so that the liquid in the centrifugal equipment is finally in a rotating state. When the viscous resistance acts on cells, the cells are sheared, and the cells are easily damaged under the shear stress, thus affecting the survival rate of cells.

### Summary

The application provides a cell centrifugation apparatus and a cell centrifugation, washing and culture method, aiming at solving the problem that the existing centrifugation equipment is likely to cause damage to cells due to shear stress in centrifugation process.

The application provides a cell centrifugation apparatus, including a centrifuge chamber and one or more liquid pushing plates; the centrifuge chamber has a rotation axis, and is used for connecting with a rotation drive mechanism, and the centrifuge chamber rotates around its rotation axis under drive of the rotation drive mechanism for centrifuging a blood sample contained in the centrifuge chamber; and the liquid pushing plates are arranged in the centrifuge chamber; when the centrifuge chamber rotates, the liquid pushing plate is used to push the blood sample to rotate synchronously along a same rotation direction as the centrifuge chamber.

According to the cell centrifugation apparatus provided by the application, one end of the liquid pushing plate is close to the rotation axis, and another end extends towards an inner side wall of the centrifuge chamber; a projection of a first side of the liquid pushing plate on a plane where a chamber bottom of the centrifuge chamber is located is a straight line or an arc, and a second side of the liquid pushing plate is perpendicular to the chamber bottom of the centrifuge chamber; and under the condition that the projection of the first side of the liquid pushing plate on the plane where the chamber bottom of the centrifuge chamber is located is a straight line, the first side of the liquid pushing plate extends in a radial direction of the centrifuge chamber.

According to the cell centrifugation apparatus provided by the application, a first gap is arranged between one end of the liquid pushing plate close to the inner side wall of the centrifuge chamber and the inner side wall; one end of the liquid pushing plate close to the chamber bottom of the centrifuge chamber is attached to the chamber bottom.

According to the cell centrifugation apparatus provided by the application, when the number of the liquid pushing plates is plural, a plurality of the liquid pushing plates are circumferentially and uniformly distributed relative to the rotation axis.

According to the cell centrifugation apparatus provided by the application, further including a chamber lid; the chamber lid is matched with an opening of the centrifuge chamber; a first liquid-through structure is arranged on the chamber lid, and a second liquid-through structure is arranged on the centrifuge chamber; a first end of the first liquid-through structure is arranged on an outer side of the chamber lid and distributed along the rotation axis, and a second end of the first liquid-through structure is arranged on an inner side of the chamber lid; and the second liquid-through structure includes a liquid-through tube and a liquid-through passage; the liquid-through tube is arranged in the centrifuge chamber; a first end of the liquid-through tube is communicated with the second end of the first liquid-through structure, and a second end of the liquid-through tube is arranged at the chamber bottom of the centrifuge chamber; the liquid-through passage is arranged in a shell wall of the chamber bottom, a first end of the liquid-through passage is communicated with the second end of the liquid-through tube, and a second end of the liquid-through passage is arranged on an inner side of the chamber bottom and communicated with an inner cavity of the centrifuge chamber.

According to the cell centrifugation apparatus provided by the application, the shell wall of the chamber bottom of the centrifuge chamber is internally provided with a third liquid-through structure; a first end of the third liquid-through structure is arranged on an outer side of the chamber bottom and distributed along the rotation axis, and a second end of the third liquid-through structure is arranged on the inner side of the chamber bottom and is communicated with the inner cavity of the centrifuge chamber.

According to the cell centrifugation apparatus provided by the application, the first liquid-through structure includes a connection tube; the connection tube and the liquid-through tube are distributed along the rotation axis; the connection tube is internally provided with a liquid passage and a gas passage, a first end of the liquid passage is provided with a liquid connection opening, and a second end of the liquid passage is communicated with the first end of the liquid-through tube; a first end of the gas passage is provided with a gas connection opening, and a second end of the gas passage is communicated with the inner cavity of the centrifuge chamber.

According to the cell centrifugation apparatus provided by the application, the chamber lid is provided with an air hole; at least one air hole is provided, and a gas permeable membrane is arranged in the air hole.

The application also provides a cell centrifugation method of the cell centrifugation apparatus, which includes the following steps: connecting the centrifuge chamber with the rotation drive mechanism, and adding the blood sample to the centrifuge chamber; and turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

The application also provides a cell washing method of the cell centrifugation apparatus, which includes the following steps: when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation; and after a centrifugation time of the blood sample reaches a preset time, extracting the liquid in the centrifuge chamber through the second liquid-through structure, so as to discharge a waste liquid after cell washing.

The application also provides a cell culture method of the cell centrifugation apparatus, which includes the following steps: adding a cell suspension with cells to be cultured into the centrifuge chamber, adjusting cell density of the cell suspension to a first preset density, and adding antibodies or magnetic beads into the centrifuge chamber to activate the cells; inputting carbon dioxide gas with a preset concentration and a culture medium with a preset components into the centrifuge chamber, and placing the centrifuge chamber containing the cell suspension and the culture medium in a preset constant temperature environment for cell culture; and detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.

The application provides a cell centrifugation apparatus and a cell centrifugation, washing and culture method. By arranging a liquid pushing plate in the centrifuge chamber, when the centrifuge chamber rotates around its axis, the blood sample in the centrifuge chamber rotates synchronously with the centrifuge chamber under the push of the liquid pushing plate. That is, there is no relative movement between the outer cells near the inner side wall of the centrifuge chamber and the inner cells near the rotation axis in the rotation direction of the centrifuge chamber. The power source of the inner cell rotation is no longer the viscous resistance between the outer cell and the inner cell, but the pushing force of the liquid pushing plate. In this way, the shearing effect on the cells is avoided when viscous resistance acts on the cells, and the damage of the cells due to the shear stress during centrifugation is reduced.

### Brief description of the drawings

In order to explain the technical solution of the embodiments of this application more clearly, the drawings described in the description of the embodiments of this application will be briefly introduced below. Obviously, the drawings in the present application and their accompanying detailed description are directed to merely exemplary embodiments of the application. F or those of ordinary skill in this field, other drawings may be obtained according to these drawings without any creative effort.
Fig. 1 is a structural schematic diagram of a cell centrifugation apparatus provided by the present application.
Fig. 2 is a schematic diagram of a force exerted on the cells during centrifugation provided by the present application.
Fig. 3 is a force diagram of cell centrifugation when a liquid pushing plate is provided by the present application.
Fig. 4 is a schematic diagram of the first arrangement structure of the liquid pushing plate in the centrifuge chamber provided by the present application.
Fig. 5 is a schematic diagram of the second arrangement structure of the liquid pushing plate in the centrifuge chamber provided by the present application.
Fig. 6 is a schematic plan view of the cell centrifugation apparatus provided by the present application.
Fig. 7 is a schematic view of the first cross-sectional structure of Fig. 6 in the direction A-A provided by the present application.
Fig. 8 is a schematic view of the second cross-sectional structure of Fig. 6 in the direction A-A provided by the present application.
Fig. 9 is a structural schematic diagram of a chamber lid provided by the present application.
Fig. 10 is another structural schematic diagram of a chamber lid provided by the present application.
Fig. 11 is a schematic flow chart of a cell centrifugation method provided by the present application.
Fig. 12 is a schematic flow chart of a cell washing method provided by the present application.
Fig. 13 is a schematic flow chart of a cell culture method provided by the present application.
Fig. 14 is a line chart of the number of cells in a cell culture process provided by the present application.

Reference signs in the figures are as follow.
1: centrifuge chamber; 11: Second liquid-through structure; 11: Liquid-through tube;
112: Liquid-through passage; 2: Liquid pushing plate; 21: Pin shaft;
3: chamber lid; 31: First liquid-through structure; 311: Connection tube;
3111: Liquid passage; 3112: Gas passage; 3113: Liquid connection opening;
3114: Gas connection opening; 32: Air hole; 33: Top cover;
34: Rim; 4: Rotation drive mechanism; 5: Observation hole.

### Detailed description of preferred embodiments

In order to make the purpose, technical solutions and beneficial effects of the present application more clear, the technical solutions in the embodiments of the application will be clearly and completely described below with reference to the drawings in the emb odiments of this application. Obviously, the described embodiments are part of the embodiments of this application, but not all of them. Based on the embodiments in this application, all other embodiments obtained by those of ordinary skill in the art without creative effort belong to the protection scope of this application.

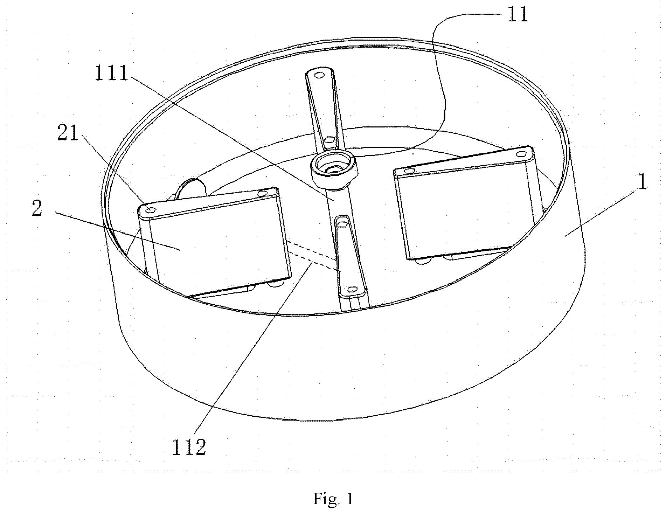

Hereinafter, a cell centrifugation apparatus and a cell centrifugation, washing and culture method provided by the present application will be described with reference to Figs. 1 to 14.

As shown in Fig. 1 to Fig. 10, this embodiment provides a cell centrifugation apparatus, which includes a centrifuge chamber 1 and one or more liquid pushing plates 2; the centrifuge chamber a has a rotation axis, and is used for connecting with a rotation drive mechanism 4, and the centrifuge chamber 1 rotates around its rotation axis under drive of the rotation drive mechanism 4 for centrifuging a blood sample contained in the centrifuge chamber 1; and the liquid pushing plates 2 are arranged in the centrifuge chamber; when the centrifuge chamber 1 rotates, the liquid pushing plate 2 is used to push the blood sample to rotate synchronously along a same rotation direction as the centrifuge chamber 1.

Specifically, in this embodiment, the liquid pushing plate 2 is arranged in the centrifuge chamber 1, so that the blood sample in the centrifuge chamber 1 rotates synchronously with the centrifuge chamber 1 under the push of the liquid pushing plate 2. That is, in the rotation direction of centrifuge chamber 1, there is no relative movement between the outer cells near the inner side wall of centrifuge chamber 1 and the inner cells near the rotation axis. The power source of the inner cell rotation is no longer the viscous resistance between the outer cell and the inner cell, but the pushing force of the liquid pushing plate. In this way, the shearing effect on the cells is avoided when viscous resistance acts on the cells, and the damage of the cells due to the shear stress during centrifugation is reduced.

It should be noted that the blood sample shown in this embodiment contains the target cells to be extracted, and the blood sample is centrifuged by the centrifugal apparatus, so that the target cells and other particles in the blood sample are layered. Thereby the target cells are extracted.

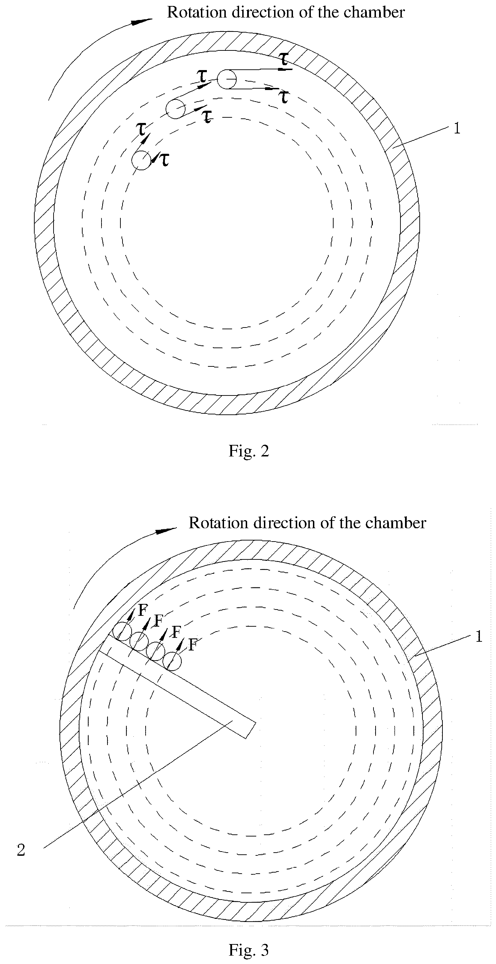

As shown in Fig. 2, the centrifuge chamber 1 shown in this embodiment performs centrifugation on the blood sample when the liquid pushing plate 2 is NOT provided. The dotted line is the movement track of cells in the blood sample. The outer cells near the inner side wall of the centrifuge chamber 1 use the viscous resistance between liquids to drive the inner cells near the rotation axis of centrifuge chamber 1 to rotate. In this process, the rotation of the inner cells is delayed to a certain extent, i.e., there is relative movement between the outer cells and the inner cells in the rotation direction of centrifuge chamber 1. In the case of relative movement, both the outer cells and inner cells are subjected to shear stress τ, and the direction of the shear stress τ is along the tangent direction of the motion trajectory. Moreover, the shear stress τ of the outer cells is larger than that of the inner cells, and the shear stress τ will cause shear damage to the cells when the shear stress τ acts on the cells. In addition, taking one of the cells as an example, the blood flow velocity of the cell near the inner side wall is higher, and the blood flow velocity of the cell near the rotation axis is lower. Hence, the cell is also subjected to a radial pressure, which will also cause shear damage to the cell when it acts on the cell.

As shown in Fig. 3, the centrifuge chamber 1 shown in this embodiment performs centrifugation on the blood sample when the liquid pushing plate 2 is provided. The dotted line is the movement track of cells in the blood sample. The outer cells and the inner cells rotate synchronously under the pushing force F of the liquid pushing plate. The power source of the inner cell rotation is no longer the viscous resistance between the outer cell and the inner cell, so the rotation of the inner cells would not be delayed, i.e., there is no relative motion between the outer cells and the inner cells in the rotation direction of centrifuge chamber 1. Without relative motion, the outer cells and the inner cells are not subjected to the shear stress, i.e., there is no shear damage to the cells during centrifugation.

It should be noted that the specific number of liquid pushing plates 2 may be 1, 2, 3 or 4, etc., and is not specifically limited here.

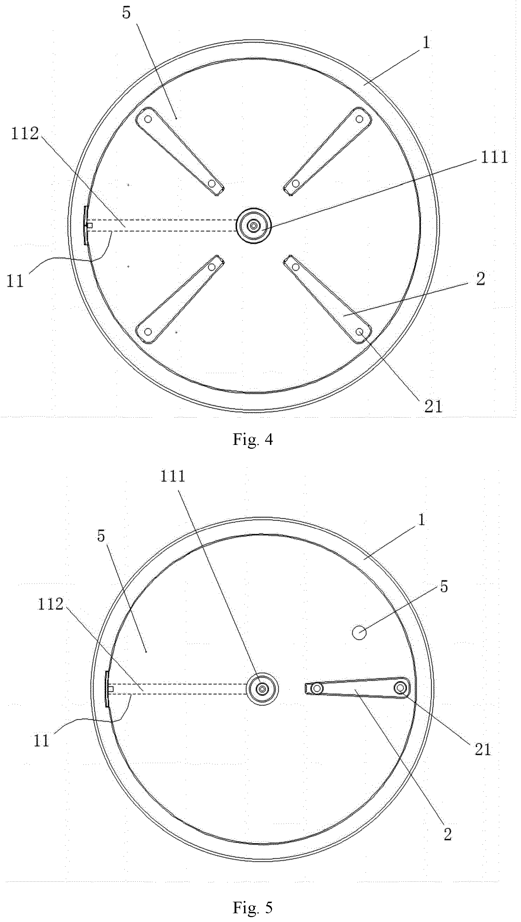

Preferably, as shown in Figs. 4 and 5, one end of the liquid pushing plate 2 is close to the rotation axis, and another end extends towards an inner side wall of the centrifuge chamber 1; a projection of a first side of the liquid pushing plate 2 on a plane where a chamber bottom of the centrifuge chamber 1 is located is a straight line or an arc, and a second side of the liquid pushing plate 2 is perpendicular to the chamber bottom of the centrifuge chamber 1

Specifically, when the projection of the first side of the liquid pushing plate 2 on the plane where the chamber bottom of the centrifuge chamber 1 is located is a straight line, the surface of the liquid pushing plate 2 for driving the blood sample to rotate is a plane; when the projection of the first side of liquid pushing plate 2 on the plane where the chamber bottom of centrifuge chamber 1 is located is an arc, the surface of the liquid pushing plate 2 for driving the blood sample to rotate is arc-shaped. According to different blood sample concentrations, the corresponding shape of liquid pushing plate is selected to ensure a good centrifugation effect for the blood sample.

Preferably, the liquid pushing plate 2 shown in this embodiment is fixedly connected with the chamber bottom of the centrifuge chamber 1, or the liquid pushing plate 2 is detachably connected with the chamber bottom of the centrifuge chamber 1.

Specifically, the liquid pushing plate 2 and the chamber bottom of the centrifuge chamber 1 are detachably connected, which is convenient for cleaning the centrifuge chamber 1 and replacing the liquid pushing plate 2.

The fixed connection shown in this embodiment includes bonding. The detachable connection includes pin shaft connection or bolt connection. Or the chamber bottom of centrifuge chamber 1 is provided with a clamping structure, and the liquid pushing plate is clamped into the clamping structure.

In one embodiment, as shown in Fig. 1, Fig. 4, Fig. 5, Fig. 7 and Fig. 8, the liquid pushing plate 2 is connected with the chamber bottom through a pin shaft 21, the liquid pushing plate 2 is provided with a pin shaft hole along the rotation axis direction, and the chamber bottom is provided with a fixing hole. One end of the pin shaft 21 is connected with the pin shaft hole, and the other end of the pin shaft 21 is connected with the fixing hole to realize detachable connection between the liquid pushing plate 2 and the chamber bottom. In order to ensure the stability of the connection, two pin shaft holes are provided, and the fixing holes are arranged in one-to-one correspondence with the pin shaft holes.

In another embodiment, the chamber bottom is provided with a first clamping groove along the radial direction of the centrifuge chamber 1, the liquid pushing plate 2 is clamped into the first clamping groove, and the liquid pushing plate 2 and the first clamping groove are in interference fit. Or, the inner side wall of the centrifuge chamber 1 is provided with a second clamping groove along the rotation axis direction, the liquid pushing plate 2 is clamped into the second clamping groove, and the liquid pushing plate 2 and the second clamping groove are in interference fit. Or, the chamber bottom and the inner side wall of the centrifuge chamber 1 are respectively provided with a first clamping groove and a second clamping groove, the first clamping groove and the second clamping groove are arranged in one-to-one correspondence, the liquid pushing plate 2 is clamped into both the first clamping groove and the second clamping groove, and the liquid pushing plate 2 is in interference fit with the first clamping groove and the second clamping groove.

Preferably, as shown in Fig. 4, Fig. 7 and Fig. 8, a first gap is arranged between one end of the liquid pushing plate 2 close to the inner side wall of the centrifuge chamber 1 and the inner side wall; one end of the liquid pushing plate 2 close to the chamber bottom of the centrifuge chamber 1 is attached to the chamber bottom.

Specifically, during the centrifugation of centrifuge chamber 1, the target cells in the blood sample gradually approach the inner side wall of centrifuge chamber 1 and finally adhere to the inner side wall of centrifuge chamber 1, so that the target cells can pass through the first gap to avoid the cells from being concentrated and deposited together. Meanwhile, the blood sample can easily pass through the first gap, ensuring that the liquid levels on both sides of the liquid pushing plate 2 are the same.

It should be noted that the first gap should not be set too large. When the first gap is too large, the liquid pushing plate 2 cannot achieve a good effect of pushing liquid. If the ratio of the first gap to the inner radius of the centrifuge chamber 1 is in the range of 1/600-1/20, the ratio of the first gap to the inner radius of the centrifuge chamber 1 may be 1/600, 1/300, 1/100, 1/60, 1/40 or 1/20, with 1/60 being preferred.

Preferably, as shown in Fig. 1, Fig. 4 and Fig. 5, a second gap is provided between the rotation axis and one end of the liquid pushing plate 2 near the rotation axis.

Specifically, by arranging the second gap between the liquid pushing plate 2 and the rotation axis, a certain operating space is provided for assembly and disassembly of the liquid pushing plate 2. Meanwhile, the linear velocity of the inner cells near the rotation axis is low during centrifugation, the second gap has little influence on the centrifugation effect of blood samples.

It should be noted that if the ratio of the second gap to the inner radius of centrifuge chamber 1 is in the range of 1/200-1/5, the ratio of the second gap to the inner radius of centrifuge chamber 1 may be 1/200, 1/100, 1/60, 1/30, 1/10 or 1/5, with 1/30 being preferred.

Preferably, when the number of the liquid pushing plates 2 is plural, a plurality of the liquid pushing plates 2 are circumferentially and uniformly distributed relative to the rotation axis.

Specifically, the centrifuge chamber 1 is divided into a plurality of relatively separated centrifugal areas by a plurality of liquid pushing plates 2. By distributing the liquid pushing plate 2 evenly along the circumferential direction of the rotation axis, it is ensured that the blood samples in each centrifugation area have the same centrifugation effect.

In one embodiment, there are two liquid pushing plates 2, so the included angle between the two liquid pushing plates 2 is 180°.

In another embodiment, there are three liquid pushing plates 2, so the included angle between two adjacent liquid pushing plates 2 is 120°.

In another embodiment, as shown in Figs. 1 and 4, there are four liquid pushing plates 2, so the included angle between two adjacent liquid pushing plates 2 is 90°.

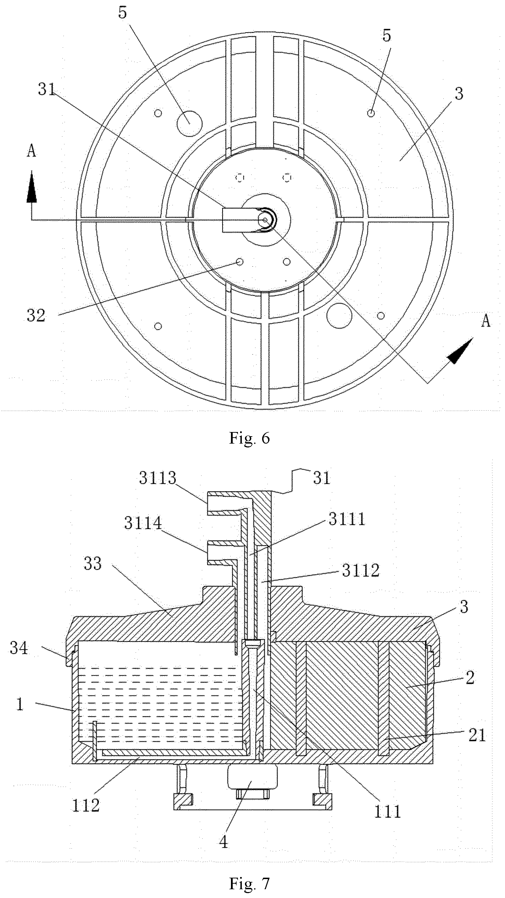

Preferably, as shown in Figs. 6, 7 and 8, the centrifugation apparatus shown in this embodiment further includes a chamber lid 3; the chamber lid 3 is matched with an opening of the centrifuge chamber 1; a first liquid-through structure 31 is arranged on the chamber lid 3, and a first end of the first liquid-through structure 31 is arranged on an outer side of the chamber lid 3 and distributed along the rotation axis, and a second end of the first liquid-through structure 31 is arranged on an inner side of the chamber lid 3.

Specifically, by setting the first liquid-through structure 31 on the chamber lid 3, blood samples can be added to the centrifuge chamber 1 without opening the chamber lid 3.

Preferably, as shown in Figs. 1, 4, 5, 7 and 8, the centrifuge chamber 1 shown in this embodiment is provided with a second liquid-through structure 11. The second liquid-through structure 11 includes a liquid-through tube 111 and a liquid-through passage 112; the liquid-through tube 111 is arranged in the centrifuge chamber 1; a first end of the liquid-through tube 111 is communicated with the second end of the first liquid-through structure 31, and a second end of the liquid-through tube 111 is arranged at the chamber bottom of the centrifuge chamber 1; the liquid-through passage 112 is arranged in a shell wall of the chamber bottom, a first end of the liquid-through passage 112 is communicated with the second end of the liquid-through tube 111, and a second end of the liquid-through passage 112 is arranged on an inner side of the chamber bottom and communicated with an inner cavity of the centrifuge chamber 1.

Specifically, in order to prevent the blood sample from splashing when it is added into the centrifuge chamber 1 through the first liquid-through structure 31, a second liquid-through structure 11 is provided. After passing through the first liquid-through structure 31, the blood sample enters the liquid-through tube 111, then the liquid-through passage 112, and finally enters the inner cavity of the centrifuge chamber 1 from the chamber bottom, thus ensuring the stability of the blood sample in the injection process. Meanwhile, the liquid-through passage 112 is buried in the shell wall of the chamber bottom to avoid the liquid-through passage 112 occupying the effective space of the inner cavity of the centrifuge chamber 1. In addition, after the blood sample is centrifuged, a cell suspension is obtained, and the cell suspension can be extracted through the second liquid-through structure 11 and the first liquid-through structure 31.

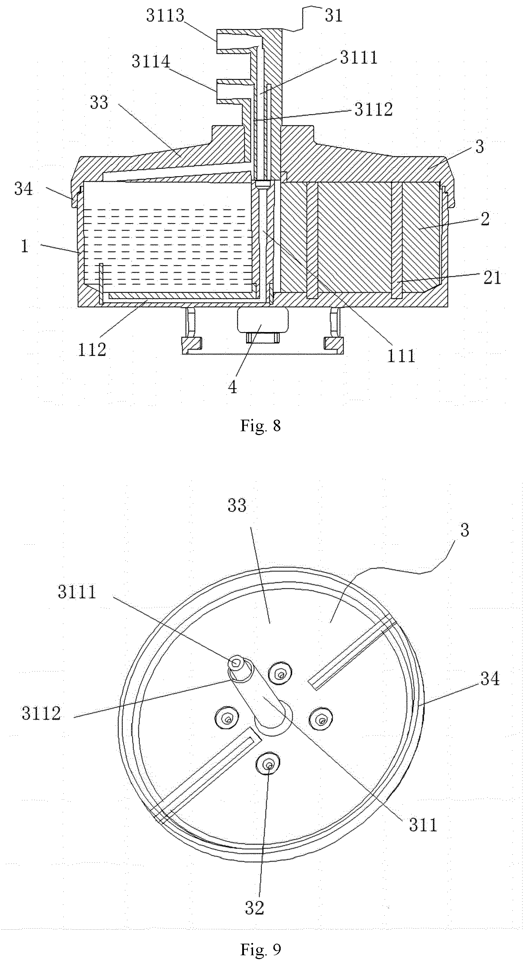

In one embodiment, as shown in Figs. 7 and 8, the liquid-through tube 111 is preferably arranged along the rotation axis to improve the stability of the centrifuge chamber 1 during rotation.

Preferably, as shown in Figs. 7 and 8 of this embodiment, one end of the liquid pushing plate 2 close to the chamber lid 3 is attached to the chamber lid 3.

Specifically, in the process of blood sample centrifugation, the arrangement of "one end of the liquid pushing plate 2 close to the chamber lid 3 is attached to the chamber lid 3" can effectively prevent the blood sample on one side of the liquid pushing plate 2 from entering the other side of liquid pushing plate 2 through the end of the liquid pushing plate 2 near the chamber lid 3.

Preferably, as shown in this embodiment, the shell wall of the centrifuge chamber 1 is internally provided with a third liquid-through structure; a first end of the third liquid-through structure is arranged on an outer side of the chamber bottom and distributed along the rotation axis, and a second end of the third liquid-through structure is arranged on the inner side of the chamber bottom and is communicated with the inner cavity of the centrifuge chamber 1.

Specifically, the blood sample can be injected into the inner cavity of centrifuge chamber 1 from the chamber bottom through the third liquid-through structure, and correspondingly, the cell suspension can be extracted through the third liquid-through structure.

Preferably, as shown in Figs. 7, 8 and 9, the first liquid-through structure 31 shown in this embodiment includes a connection tube 311; the connection tube 311 and the liquid-through tube 111 are distributed along the rotation axis; the connection tube 311 is internally provided with a liquid passage 3111 and a gas passage 3112, a first end of the liquid passage 3111 is provided with a liquid connection opening 3113, and a second end of the liquid passage 3111 is communicated with the first end of the liquid-through tube 111; a first end of the gas passage 3112 is provided with a gas connection opening 3114, and a second end of the gas passage 3112 is communicated with the inner cavity of the centrifuge chamber 1.

Specifically, the centrifuge chamber 1 shown in this embodiment is used in cell culture. Without opening the chamber lid 3, culture medium can be injected into the centrifuge chamber 1 through the liquid connection opening 3113, and carbon dioxide with a certain concentration can be injected into the centrifuge chamber 1 through the gas connection opening 3114.

It should be noted that the liquid passage 3111 may be used not only for introducing liquid, but also for introducing gas. Similarly, the gas passage 3112 may be used not only for introducing gas, but also for introducing liquid. The liquid passage 3111 and gas passage 3112 are separated from each other. When one of the liquid passage 3111 and gas passage 3112 is filled with liquid, the other one can be filled with gas at the same time. The two processes of introducing liquid and gas do not interfere with each other.

In one embodiment, the liquid passage 3111 and the gas passage 3112 are arranged side by side.

In another embodiment, as shown in Fig. 7 and Fig. 9, the liquid passage 3111 is sleeved in the gas passage 3112, and the second end of the gas passage 3112 is arranged along the rotation axis and communicates with the inner cavity of the centrifuge chamber 1.

In another embodiment, as shown in Fig. 8, the second end of the gas passage 3112 extends in the radial direction of the centrifuge chamber 1 and communicates with the inner cavity of the centrifuge chamber 1.

Preferably, as shown in Fig. 6, Fig. 9 and Fig. 10, the chamber lid 3 shown in this embodiment is provided with an air hole 32, at least one air hole 32 is provided, and a gas permeable membrane is arranged in the air hole 32.

Specifically, when the centrifuge chamber 1 shown in this embodiment is used for cell culture, the gas permeable membrane can prevent external bacteria from entering the centrifuge chamber 1. And at the same time, the gas permeable membrane can realize gas exchange between the centrifuge chamber 1 and the outside, thus ensuring the regular respiration of cells in the centrifuge chamber 1.

It should be noted here that the gas permeable membrane is preferably a hydrophobic gas permeable membrane.

Preferably, as shown in Fig. 7, Fig. 8, Fig. 9 and Fig. 10, the chamber lid 3 shown in this embodiment includes a top cover 33 and a rim 34, the rim 34 extends along the circumferential direction of the top cover 33, the chamber lid 3 covers the opening of the centrifuge chamber 1, and the inner side of the rim 34 is attached to the outer side wall of the centrifuge chamber 1.

Specifically, with the arrangement of "the chamber lid 3 covers the opening of the centrifuge chamber 1, and the inner side of the rim 34 is attached to the outer side wall of the centrifuge chamber 1", the airtightness between the chamber lid 3 and the centrifuge chamber 1 is ensured.

Preferably, as shown in Fig. 4 to Fig. 6, in order to conveniently observe the cell culture state in the centrifuge chamber 1 when the cells are cultured with the centrifuge chamber 1, a plurality of observation hole 5 are arranged on the centrifuge chamber 1 or the chamber lid 3. In the process of cell culture, observation hole 5 is in a normally closed state. To observe, open the observation hole 5 and connect the observation hole 5 with an optical monitoring equipment for observation.

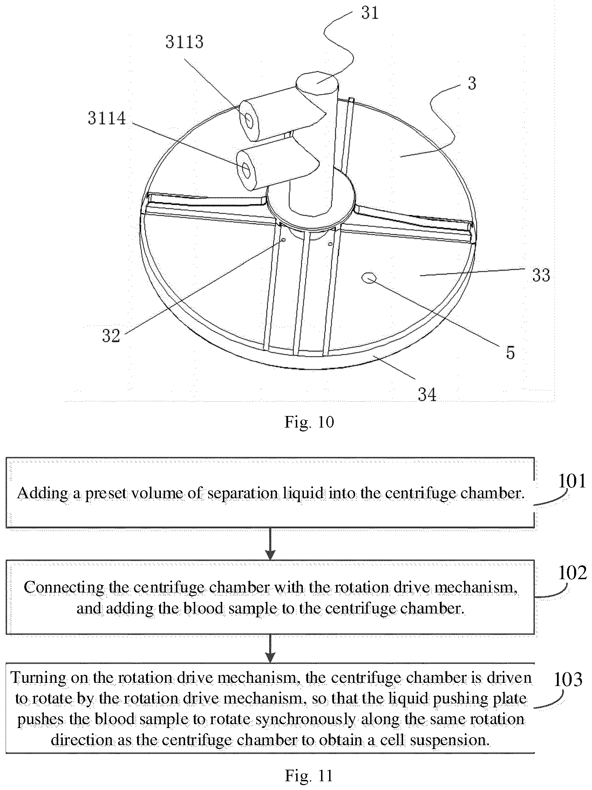

Preferably, as shown in Fig. 11, the embodiment also provides a cell centrifugation method of a cell centrifugation apparatus, including the following steps.

Step 102: connecting the centrifuge chamber with the rotation drive mechanism, and adding the blood sample to the centrifuge chamber.

Step 103: turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

Preferably, before Step 102, it also includes Step 101: adding a preset volume of separation liquid into the centrifuge chamber.

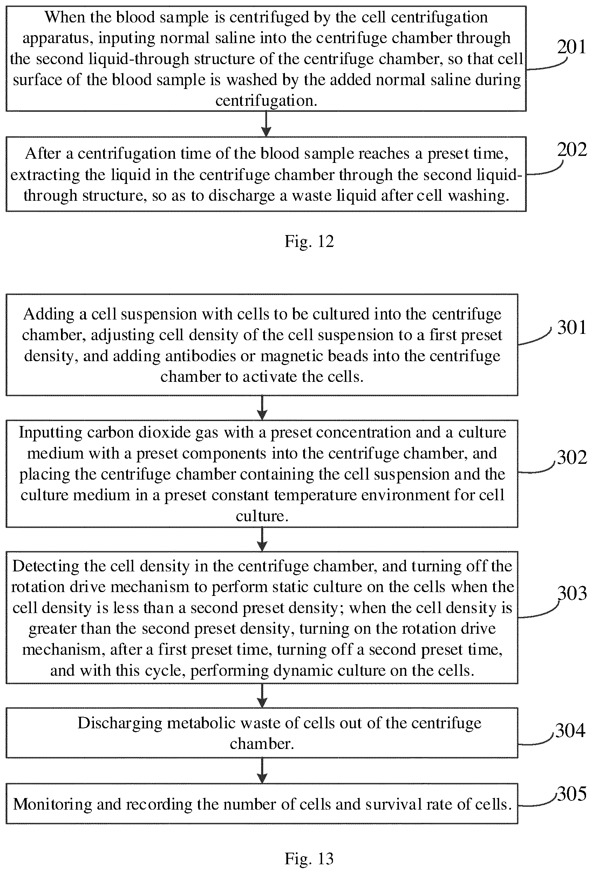

Preferably, as shown in Fig. 12, the embodiment also provides a cell washing method of a cell centrifugation apparatus, including the following steps.

Step 201: when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation.

Step 202: after a centrifugation time of the blood sample reaches a preset time, extracting the liquid in the centrifuge chamber through the second liquid-through structure, so as to discharge a waste liquid after cell washing.

Specifically, the centrifuge chamber shown in this embodiment can wash cells during centrifugation, which improves the efficiency of cleaning, and the cells obtained after cleaning are used for culture.

Preferably, as shown in Fig. 13, this embodiment also provides a cell culture method of a cell centrifugation apparatus, including the following steps.

Step 301: adding a cell suspension with cells to be cultured into the centrifuge chamber, adjusting cell density of the cell suspension to a first preset density, and adding antibodies or magnetic beads into the centrifuge chamber to activate the cells.

Step 302: inputting carbon dioxide gas with a preset concentration and a culture medium with a preset components into the centrifuge chamber, and placing the centrifuge chamber containing the cell suspension and the culture medium in a preset constant temperature environment for cell culture.

Step 303: detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.

It should be noted that the preset concentration of carbon dioxide ranges from 2% to 7%, which may specifically be 2%, 3%, 4%, 5%, 6% or 7%. The range of constant temperature is 0-40°C, which may specifically be 0°C, 10°C, 19°C, 20°C, 30°C, 37°C or 40°C. According to the characteristics of different cells, the temperature of constant temperature environment should be adjusted accordingly.

Preferably, after Step 303 shown in this embodiment, it further includes:
Step 304: discharging metabolic waste of cells out of the centrifuge chamber.
Step 305: monitoring and recording the number of cells and survival rate of cells.

Specifically, in dynamic culture, the liquid pushing plate can effectively stir the culture medium, so that the cells can be fully mixed with the culture medium. Meanwhile, the liquid pushing plate can also effectively stir the cells. Thereby preventing the cells from being concentrated and deposited together, and improving the cell culture effect.

It should be pointed out that the preset components of the culture medium include inorganic salts, carbon sources, nitrogen sources, interleukin and trace elements. After the cells are cultured for a period of time, the cell density increases, so the first preset density is smaller than the second preset density. The first preset density ranges from 0.2×10⁶ cells/ml to 2.0×10⁶ cells/ml, and the second preset density ranges from 2.0×10⁶ cells/ml to 5.0×10⁶ cells/ml. The specific method of adjusting the cell density in the cell suspension to the first preset density is: adding culture medium to the cell suspension to dilute the cell density in the cell suspension to the first preset density.

It should also be noted that the first preset time is preferably 1s and the second preset time is preferably 6s. In this case, the dynamic culture process is as follows: one cycle period of the centrifuge chamber is 7s; the centrifuge chamber rotates for 1s and then stops for 6s. The specific values of the first preset time and the second preset time are not limited here, and may be adaptively adjusted according to the cell density.

Preferably, in order to compare the influence of cell centrifugation on cell culture results with and without the liquid pushing plate. A control experiment was conducted in the embodiment. There were two control groups, each with five experimental samples.

In the first control group, cells were centrifuged with the liquid pushing plate, and in the second control group, cells were centrifuged without the liquid pushing plate.

After the centrifugation was completed, the cell yield of each experimental sample in the two control groups was calculated respectively. The results are shown in Table 1.

**Table 1 Statistical table of cell yield after centrifugation:**

| | Sample 1 | Sample 2 | Sample 3 | Sample 4 | Sample 5 | Average |
|---|---|---|---|---|---|---|
| First control group | 96.1% | 94.6% | 93.6% | 96.4% | 95.6% | 95.3% |
| Second control group | 81.6% | 76.2% | 75.4% | 84.3% | 80.0% | 79.5% |

It can be seen that the cell yield of the first control group is much higher than that of the second control group. Because there is no liquid pushing plate in the second control group, a large number of cells are damaged by shearing during centrifugation, which reduces the cell yield.

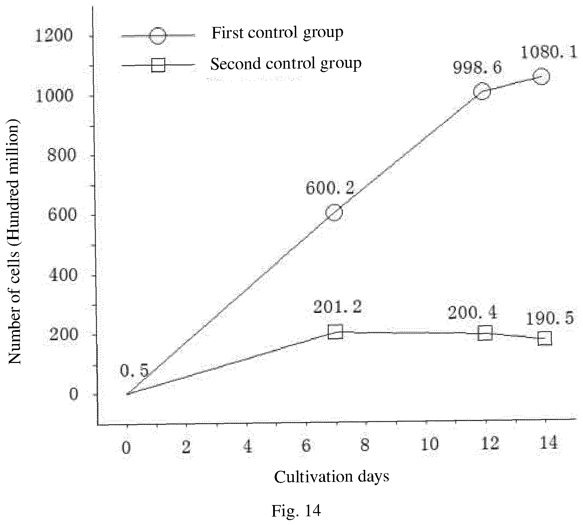

After centrifugation, the cells were further cultured. The numbers of the initial cultured cells in the first control group and the second control group were both 50 million, and the number of cells was calculated every day. The statistical results are shown in Fig. 14.

On the 14th day, the statistics showed that the number of cells in the first control group increased from 50 million to 108 billion.

On the 7th day, the statistics showed that the number of cells in the second control group increased from 50 million to 20 billion. On the 12th day, the statistics showed that the number of cells in the second control group was 20.05 billion. On the 14th day, the number of cells in the second control group decreased to 19.05 billion.

Therefore, it can be concluded that after centrifugation, although the cell yield of the second control group was about 83% of that of the first control group, a large number of cells in the second control group were damaged to varying degrees due to shearing, and the biological activity of the cells damaged by shearing decreased, so they could not be cultured normally. It can be seen that by setting the liquid pushing plate in the centrifuge chamber, the damage of cells due to shearing during centrifugation can be effectively reduced, thus ensuring the cell culture effect.

Finally, it should be noted that the above embodiments are only used to illustrate the technical solutions of the present application, but not to limit it. Although the present application has been described in detail with reference to the foregoing embodiments, those skilled in the art would understand that it is possible to modify the technical solutions described in the foregoing embodiments, or to replace some technical features with equivalents. However, these modifications or substitutions do not make the essence of the corresponding technical solutions deviate from the spirit and scope of the technical solutions of various embodiments of the present application. Further, unless otherwise required by context, singular terms shall include pluralities and plural terms shall include the singular_{∘}

### Industrial applicability

The application provides a cell centrifugation apparatus and a cell centrifugation, washing and culture method. The cell centrifugation apparatus includes a centrifuge chamber and one or more liquid pushing plates; the centrifuge chamber has a rotation axis, and is used for connecting with a rotation drive mechanism, and the centrifuge chamber rotates around its rotation axis under drive of the rotation drive mechanism for centrifuging a blood sample contained in the centrifuge chamber; and the liquid pushing plates are arranged in the centrifuge chamber; when the centrifuge chamber rotates, the liquid pushing plate is used to push the blood sample to rotate synchronously along a same rotation direction as the centrifuge chamber. According to the application, the liquid pushing plate is arranged in the centrifuge chamber, and during the rotation of the centrifuge chamber, the blood sample in the centrifuge chamber rotates synchronously with the centrifuge chamber under the pushing force of the liquid pushing plate, so that the cells in the blood sample are prevented from being subjected to shearing during centrifugation, and cell damage due to the shear stress during the centrifugation process is reduced. Hence, the present application has good economic value and application prospect.

## Claims

1. A cell centrifugation apparatus, comprising a centrifuge chamber and one or more liquid pushing plates;
the centrifuge chamber has a rotation axis, and is used for connecting with a rotation drive mechanism, and the centrifuge chamber rotates around its rotation axis under drive of the rotation drive mechanism for centrifuging a blood sample contained in the centrifuge chamber; and
the liquid pushing plates are arranged in the centrifuge chamber; when the centrifuge chamber rotates, the liquid pushing plate is used to push the blood sample to rotate synchronously along a same rotation direction as the centrifuge chamber.

2. The cell centrifugation apparatus of claim 1, wherein,
one end of the liquid pushing plate is close to the rotation axis, and another end extends towards an inner side wall of the centrifuge chamber;
a projection of a first side of the liquid pushing plate on a plane where a chamber bottom of the centrifuge chamber is located is a straight line or an arc, and a second side of the liquid pushing plate is perpendicular to the chamber bottom of the centrifuge chamber; and
under the condition that the projection of the first side of the liquid pushing plate on the plane where the chamber bottom of the centrifuge chamber is located is a straight line, the first side of the liquid pushing plate extends in a radial direction of the centrifuge chamber.

3. The cell centrifugation apparatus of claim 2, wherein,
a first gap is arranged between one end of the liquid pushing plate close to the inner side wall of the centrifuge chamber and the inner side wall; one end of the liquid pushing plate close to the chamber bottom of the centrifuge chamber is attached to the chamber bottom.

4. The cell centrifugation apparatus of claim 2, wherein,
when the number of the liquid pushing plates is plural, a plurality of the liquid pushing plates are circumferentially and uniformly distributed relative to the rotation axis.

5. The cell centrifugation apparatus of claim 3, further comprising a chamber lid;
the chamber lid is matched with an opening of the centrifuge chamber; a first liquid-through structure is arranged on the chamber lid, and a second liquid-through structure is arranged on the centrifuge chamber;
a first end of the first liquid-through structure is arranged on an outer side of the chamber lid and distributed along the rotation axis, and a second end of the first liquid-through structure is arranged on an inner side of the chamber lid; and
the second liquid-through structure comprises a liquid-through tube and a liquid-through passage; the liquid-through tube is arranged in the centrifuge chamber; a first end of the liquid-through tube is communicated with the second end of the first liquid-through structure, and a second end of the liquid-through tube is arranged at the chamber bottom of the centrifuge chamber; the liquid-through passage is arranged in a shell wall of the chamber bottom, a first end of the liquid-through passage is communicated with the second end of the liquid-through tube, and a second end of the liquid-through passage is arranged on an inner side of the chamber bottom and communicated with an inner cavity of the centrifuge chamber.

6. The cell centrifugation apparatus of claim 3, wherein,
the shell wall of the chamber bottom of the centrifuge chamber is internally provided with a third liquid-through structure;
a first end of the third liquid-through structure is arranged on an outer side of the chamber bottom and distributed along the rotation axis, and a second end of the third liquid-through structure is arranged on the inner side of the chamber bottom and is communicated with the inner cavity of the centrifuge chamber.

7. The cell centrifugation apparatus of claim 5, wherein,
the first liquid-through structure comprises a connection tube;
the connection tube and the liquid-through tube are distributed along the rotation axis;
the connection tube is internally provided with a liquid passage and a gas passage, a first end of the liquid passage is provided with a liquid connection opening, and a second end of the liquid passage is communicated with the first end of the liquid-through tube; a first end of the gas passage is provided with a gas connection opening, and a second end of the gas passage is communicated with the inner cavity of the centrifuge chamber.

8. The cell centrifugation apparatus of claim 5, wherein,
the chamber lid is provided with an air hole; and
at least one air hole is provided, and a gas permeable membrane is arranged in the air hole.

9. A cell centrifugation method based on the cell centrifugation apparatus of claim 1, comprising:
connecting the centrifuge chamber with the rotation drive mechanism, and adding the blood sample to the centrifuge chamber; and
turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

10. A cell centrifugation method based on the cell centrifugation apparatus of claim 2, comprising:
connecting the centrifuge chamber with the rotation drive mechanism, and adding the blood sample to the centrifuge chamber; and
turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

11. A cell centrifugation method based on the cell centrifugation apparatus of claim 3, comprising:
connecting the centrifuge chamber with the rotation drive mechanism, and adding the blood sample to the centrifuge chamber; and
turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

12. A cell centrifugation method based on the cell centrifugation apparatus of claim 4, comprising:
connecting the centrifuge chamber with the rotation drive mechanism, and adding the blood sample to the centrifuge chamber; and
turning on the rotation drive mechanism, the centrifuge chamber is driven to rotate by the rotation drive mechanism, so that the liquid pushing plate pushes the blood sample to rotate synchronously along the same rotation direction as the centrifuge chamber to obtain a cell suspension.

13. A cell washing method based on the cell centrifugation apparatus of claim 1, comprising:
when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation; and
after a centrifugation time of the blood sample reaches a preset time, extracting the liquid in the centrifuge chamber through the second liquid-through structure, so as to discharge a waste liquid after cell washing.

14. A cell washing method based on the cell centrifugation apparatus of claim 2, comprising:
when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation; and
after a centrifugation time of the blood sample reaches a preset time, extracting the liquid in the centrifuge chamber through the second liquid-through structure, so as to discharge a waste liquid after cell washing.

15. A cell washing method based on the cell centrifugation apparatus of claim 3, comprising:
when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation; and
after a centrifugation time of the blood sample reaches a preset time, extracting the liquid in the centrifuge chamber through the second liquid-through structure, so as to discharge a waste liquid after cell washing.

16. A cell washing method based on the cell centrifugation apparatus of claim 4, comprising:
when the blood sample is centrifuged by the cell centrifugation apparatus, inputing normal saline into the centrifuge chamber through the second liquid-through structure of the centrifuge chamber, so that cell surface of the blood sample is washed by the added normal saline during centrifugation; and
after a centrifugation time of the blood sample reaches a preset time, extracting the liquid in the centrifuge chamber through the second liquid-through structure, so as to discharge a waste liquid after cell washing.

17. A cell culture method based on the cell centrifugation apparatus of claim 1, comprising:
adding a cell suspension with cells to be cultured into the centrifuge chamber, adjusting cell density of the cell suspension to a first preset density, and adding antibodies or magnetic beads into the centrifuge chamber to activate the cells;
inputting carbon dioxide gas with a preset concentration and a culture medium with a preset components into the centrifuge chamber, and placing the centrifuge chamber containing the cell suspension and the culture medium in a preset constant temperature environment for cell culture; and
detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.

18. A cell culture method based on the cell centrifugation apparatus of claim 2, comprising:
adding a cell suspension with cells to be cultured into the centrifuge chamber, adjusting cell density of the cell suspension to a first preset density, and adding antibodies or magnetic beads into the centrifuge chamber to activate the cells;
inputting carbon dioxide gas with a preset concentration and a culture medium with a preset components into the centrifuge chamber, and placing the centrifuge chamber containing the cell suspension and the culture medium in a preset constant temperature environment for cell culture; and
detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.

19. A cell culture method based on the cell centrifugation apparatus of claim 3, comprising:
adding a cell suspension with cells to be cultured into the centrifuge chamber, adjusting cell density of the cell suspension to a first preset density, and adding antibodies or magnetic beads into the centrifuge chamber to activate the cells;
inputting carbon dioxide gas with a preset concentration and a culture medium with a preset components into the centrifuge chamber, and placing the centrifuge chamber containing the cell suspension and the culture medium in a preset constant temperature environment for cell culture; and
detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.

20. A cell culture method based on the cell centrifugation apparatus of claim 4, comprising:
adding a cell suspension with cells to be cultured into the centrifuge chamber, adjusting cell density of the cell suspension to a first preset density, and adding antibodies or magnetic beads into the centrifuge chamber to activate the cells;
inputting carbon dioxide gas with a preset concentration and a culture medium with a preset components into the centrifuge chamber, and placing the centrifuge chamber containing the cell suspension and the culture medium in a preset constant temperature environment for cell culture; and
detecting the cell density in the centrifuge chamber, and turning off the rotation drive mechanism to perform static culture on the cells when the cell density is less than a second preset density; when the cell density is greater than the second preset density, turning on the rotation drive mechanism, after a first preset time, turning off a second preset time, and with this cycle, performing dynamic culture on the cells.
